# EUROPEAN PATENT APPLICATION

(11) **EP 4 155 410 A1**
(43) Date of publication of application: **29.03.2023**
(21) Application number: 21808722.9
(22) Date of filing: 20.05.2021
(51) Int. Cl.: C12N 15/85, C12N 15/24, C12N 7/01, A61K 39/145, C07K 14/54, A61P 31/16, A61P 31/04

(54) **NUCLEIC ACID CONSTRUCT, RECOMBINANT INFLUENZA VIRUS, METHOD FOR PREPARING A RECOMBINANT INFLUENZA VIRUS, COMPOSITION AND USE**

(30) Priority: 21.05.2020 BR 102020010208
(71) Applicant: Fundação Oswaldo Cruz, 21045-900 Rio de Janeiro, RJ (BR)
(72) Inventor: MACHADO, Alexandre, de Magalhães, Vieira, 30180-090 Belo Horizonte, MG (BR); MESSIAS, Sarah, Giarola, da Silva, 31260-260 Belo Horizonte, MG (BR); GONÇALVES, Ana Paula, de Faria, 31275-120 Belo Horizonte, MG (BR); FAUSTINO, Lídia Paula, 32670-676 Betim, MG (BR); PEREIRA, Igor, A., 30260-510 Belo Horizonte, MG (BR); DE PAULA, Ianca Évelyn, Silva, 30640-105 Belo Horizonte, MG (BR); ARAÚJO, Márcio, Sobreira, Silva, 30380-530 Belo Horizonte, MG (BR); TAVARES, Luciana, Pádua, 30535-490 Belo Horizonte, MG (BR); ALVES, Pedro Augusto, 31270-720 Belo Horizonte, MG (BR); XAVIER, Marcelo, Pascoal, 30180-090 Belo Horizonte, MG (BR); CARDOSO, Kimberly, Freitas, 30190-110 Belo Horizonte, MG (BR); DE CARVALHO, Ketyllen, Reis, Andrade, 31530-330 Belo Horizonte, MG (BR)
(74) Representative: Zacco Denmark A/S
(86) International application number: PCT/BR2021/050216
(87) International publication number: WO 2021/232130

(57) **Abstract**

The present invention relates to a nucleic acid construct, a recombinant multiply-defective influenza virus, that promotes expression of an immunomodulatory protein in a host. This is applicable to the development of vaccines against infectious diseases, particularly those caused by influenza virus and Coronavirus.

## Description

### Field of Invention

The present invention is positioned in the field of vaccinology, describing a recombinant influenza virus defective for multiplication, applicable to the development of prophylactic and therapeutic vaccines against infectious diseases, particularly those caused by the influenza virus, by coronavirus or even in the field of biomedical research, as a tool to promote the production of immune modulator polypeptides in the airways.

### Background of the invention

Influenza pandemics are defined by a dramatic global increase in morbidity and mortality due to the emergence of antigenically novel influenza viruses (usually of a new subtype). Several factors combine to modulate the severity and extent of a pandemic, including the low degree of immunity in the population, the virulence of the pandemic influenza virus, and the efficiency with which the virus can be transmitted among humans. The latter is generally influenced not only by the characteristics of the virus but also by population density and ease of travel in and out within a region. The virus responsible for the pandemic is usually a newly emerged antigenic variant with which most of the population has had no previous contact and therefore has little or no immunity. The ability to multiply and be transmitted efficiently in humans are prerequisites for the rapid spread of the virus in the population.

Pandemic influenza spreads very quickly and can have devastating impact. The most severe pandemic in the 20th century, the 1918 pandemic, killed more than 500,000 American citizens and between 20 and 40 million people around the world. A pandemic can produce waves of illness, with peaks of incidence separated by several weeks to months. The relatively rapid onset and spread of pandemic influenza poses several problems for the response to a global health hazard of this magnitude and imposes overwhelming burdens on emergency responders and health care technicians. Rapid identification and response to the emerging pandemic are necessary elements to minimize its impact. Several programs are underway around the world to monitor emerging influenza viruses, including the avian influenza virus, which cause sporadic but mostly lethal infections in humans. These surveillance data are used in conjunction with predefined pandemic alert levels in order to identify the likelihood of the threat and provide guidance for effective response.

Vaccination is the most important public health measure to prevent illness caused by both annual epidemics and influenza pandemics. The short interval between the identification of a pandemic virus and its dispersal around the world, as occurred during the 2009 swine flu pandemic, poses a significant challenge to producing vaccines that are both specific to the pandemic virus and sufficient in quantity to protect a broad segment of the population. The short response times required to produce a "pandemic vaccine" do not allow for prolonged research and development processes to provide an effective response.

As a solution, the reverse genetics technique allows the rapid expression and recombinant manipulation of RNA viruses in cell culture, for particular applications on vaccine production. The method involves transfecting host cells with one or more expression constructs encoding the viral genome and subsequent recovery of the virus from the host cells.

For example, documents WO2007002008 and WO2007124327 describe a reverse genetics method in which influenza virus genomic RNA is expressed in canine cells using the canine RNA polymerase I (pol I) promoter. Other sources have reported the expression of influenza genomic RNA in human cells using the human pol I promoter.

More recently, the document WO2010133964 described a reverse genetics method to produce recombinant influenza virus in host cells using an exogenous pol I promoter from an organism of distinct taxonomic class (e.g., using the human pol I promoter in canine host cells).

However, although reverse genetics techniques ensure agility in the production of influenza vaccine strains, seasonal strains change annually according to predicted prevalence for the period. So, the next generation of influenza vaccines should confer cross-protection against different strains and subtypes of influenza virus thus generating a hetero subtypic response.

In this regard, the present invention represents an advantage over the current methodology for producing vaccines against infections caused by the various subtypes of influenza viruses. Recombinant viruses of the present invention are capable of generating long-lasting heterosubtypic responses, protecting against antigenically distinct influenza viruses even two and a half months after intranasal treatment. The technology in question also has the advantage of protecting immunized individuals against secondary bacterial infections, which are responsible for a large percentage of complicated cases and deaths in patients infected with influenza viruses. Therefore, vaccines comprising these recombinant viruses, both as immunogens and as adjuvants, will allow circumventing the problems related to the frequent antigenic incompatibility between the viruses included in the vaccine formulation and those circulating in the population, as well as mitigate problems related to secondary bacterial infections.

In addition, the present invention can be applied in the field of biomedical research as a tool to enable the topical expression of immune modulator polypeptides in the airways. Thus, the invention makes it possible to evaluate the role of these immunomodulatory proteins in the establishment, progression, and resolution of Inflammatory or infectious processes in the respiratory tract, such as those caused by influenza viruses and coronavirus.

Kang and co-workers demonstrated that administration of it the immunomodulatory proteins by the intranasal route, specifically interleukin-7 (IL-7), is able to induce a prophylactic response against subsequent influenza virus infection in mice. Complete protection is obtained when challenge with influenza virus occurs up to 14 days after nasal treatment of mice with Fc-fused IL-7 (Kang et al. "Intranasal introduction of Fc-fused interleukin-7 provides long-lasting prophylaxis against lethal influenza virus infection." Journal of virology 90.5 (2016): 2273-2284).

However, the development of an influenza vaccine requires eliciting a long-lasting heterosubtypic immune response in order to keep to protected against subsequent exposures to the infectious agent. Kang and co-workers demonstrate that intranasal administration of IL-7-Fc is able to promote an immune cant favorable for fighting respiratory infections by proliferating and activating white blood cells in the lungs. However, the strategy used by these authors is not able to generate memory response, because there is no specific antigenic stimulus and therefore no activation of cells and transcription factors necessary for cell differentiation to occur. The formation of a memory response is dependent on antigen presentation by antigen-presenting cells (APCs) (*e*.*g*. dendritic cells and macrophages) capable of activating the cells of adaptive immunity, such as T and B lymphocytes, responsible for long-lasting memory immunity (Devarajan P, et al. New Insights into the Generation of CD4 Memory May Shape Future Vaccine Strategies for Influenza. Front Immunol. 2016;7:136). In fact, the significant reduction in protection after 21 and 35 days demonstrates that the local nonspecific stimulus obtained by Kang et al. is transient, unlike what occurs in the presence of viral antigen (Jones PD, Ada GL. Influenza virus-specific antibody-secreting cells in the murine lung during primary influenza virus infection. J Virol. 1986;60(2):614-619). As already shown, the viral antigenic stimulus is critical for the formation of the lymphoid tissues associated with the bronchi and/or nasal region, BALT and NALT respectively, important for the maintenance of memory cells for long periods (Onodera T, et al. Memory B cells in the lung participate in protective humoral immune responses to pulmonary influenza virus reinfection. PNAS 2012;109(7):2485-2490).

In mice, the definition of immunological memory is considered to be that measured 50 days after inoculation (Hye Mee Joo et al. Broad dispersion and lung localization of virus-specific memory B cells induced by influenza pneumonia; Primary and long-term B-cell responses in the upper airway and lung after influenza A virus infection. PNAS 2008, 105 (9) 3485-3490). However, it is not possible to directly compare the duration of immunity in humans with that assessed in the animal model (mouse), considering mainly the differences in life span and cell population/phenotype between the two, which interfere in the kinetics and duration of the immune response (Boyden, A.W., Frickman, A.M., Legge, K.L. et al. Primary and long-term B-cell responses in the upper airway and lung after influenza A virus infection. Immunol Res 59, 73-80 (2014)).

There are no reports in the literature on what would be the propitious duration of the heterosubtypic response in humans, but several studies show that the pre-existence of T lymphocytes (CD4⁺ and/or CD⁸+) in individuals with an absence of cross-response antibodies is related to a reduction in disease symptoms, viral spread, and consequently, disease aggravation. These cells mainly recognize highly conserved internal antigens among different influenza subtypes, such as NB, PB1, M1 and M2, and although they are not able to neutralize the viral particle, they limit its replication in the airways, reducing the morbidity associated with the disease and the risk of death (Wilkinson, T., Li, C., Chui, C. et al. Preexisting influenza-specific CD4+ T cells correlate with disease protection against influenza challenge in humans. Nat Med 18, 274-280 (2012); Sridhar, et al. Cellular immune correlates of protection against symptomatic pandemic influenza. Nat Med 19, 1305-1312 (2013).; Hayward AC, et al. Natural T Cell-mediated Protection against Seasonal and Pandemic Influenza. Results of the Flu Watch Cohort Study. Am J Respir Crit Care Med. 2015;191(12):1422-1431). A recent study (2019) used ferrets, the gold standard model for human influenza infection, to analyze the cross-response induced by a previous infection. The authors demonstrated that animals infected with a low dose of influenza A H1N1 (100 PFU) were partially protected against challenge with a heterosubtypic H3N2 isolated after 28 days, with reduced symptoms and viral spread, suggesting a protection mediated by memory T lymphocytes, since no neutralizing anti-H3N2 antibodies were detected (Gooch KE, et al. Heterosubtypic cross-protection correlates with cross-reactive interferon-gamma-secreting lymphocytes in the ferret model of influenza. Sci Rep. 2019; 9(1): 2617). In another study proposing the development of a universal influenza vaccine, the authors used a recombinant virus encoding different viral proteins and the IL-15 cytokine gene as adjuvant and reported significant cross-protection against other influenza subtypes 21 days after the third dose of subcutaneous vaccination (Valkenburg et al. Protection by universal influenza vaccine is mediated by memory CD4 T cells. Vaccine, 2018, 4198-4206).

In any case, the optimal duration of heterosubtypic vaccine response in humans for the development of an influenza vaccine should be at least 2-4 months, in order to ensure the greatest protection during the season of greatest virus circulation, which occurs right after the vaccination campaigns.

Carriage of the immunomodulatory protein Il-7 by an influenza virus, as achieved by the present invention, enables the production of said protein directly in infected cells, in a transient and localized manner, resulting in the generation of heterosubtypic memory immune response. Thus, protection can be achieved against influenza viruses that are antigenically distinct from the one used in vaccination. In contrast, tone she results of Kang *et al.,* the protection obtained in the present invention is maintained even when the challenge with the virus is performed two and a half months after intranasal inoculation, demonstrating that there is induction of immunological memory. Recombinant Flu IL-7 virus has been shown to be safe and unable to cause disease in inoculated mice, there is no need for revaccination, and heterosubtypic protection was observed at later times (30 and 60 days after immunization) than the studies mentioned above.

In finally, it is of great relevance that immunization with influenza viruses encoding IL-7 was able to confer protection against secondary bacterial infection caused by *Streptococcus pneumoniae.* Secondary bacterial infections, especially those caused by *S. pneumoniae,* are responsible for the most severe complications of influenza conditions, resulting in a worse prognosis, especially in children and the elderly. Another outstanding aspect of the present invention consists of the immunomodulatory proteins acting on inflammatory or infectious processes caused by coronavirus.

The invention will be presented in more detail below.

### Detailed description invention

The invention is, in essence, a recombinant defective influenza virus for multiplication that expresses an immunomodulatory protein.

In one mode of the invention, said immunomodulatory protein can be a chemokine or a cytokine. Preferably, the said protein is a cytokine of the interleukin class, particularly interleukin 7 (IL-7).

In a particular form of the invention, recombinant influenza virus is obtained from a nucleic acid construct comprising a neuraminidase (NA) gene truncated by removal of its medial portion and into which the sequence encoding the immunomodulatory protein has been inserted.

In another mode, the nucleic acid construct additionally comprises a heterologous promoter region and terminator region. Also, in one mode of the invention, the said promoter region is the promoter region of human RNA Polymerase I (Pol I).

In another mode, the nucleic acid construcconstructsed by the invention is that described by SEQ ID NO: 1 corresponding to plasmid pPRNA166x178.

Further, in one mode, the nucleic acid construct described in the invention comprises the ribozyme terminator sequence of hepatitis delta virus; followed by (i) a truncated promoter of human Pol I; (ii) sequence encoding the first 166 nucleotides of neuraminidase, in which all ATG strands have been mutated; (iii) sequence encoding the last 178 nucleotides of neuraminidase (iv) 3' promoter of neuraminidase; (v) repetition of the last 42 nucleotides of the neuraminidase ORF; and (vi) truncated promoter of human RNA Pol I; in which a sequence encoding an immunomodulatory protein is inserted between the first 166 and the last 178 nucleotides of neuraminidase.

Another mode of the invention provides for a recombinant influenza virus comprising a truncated neuraminidase gene into which has been inserted the nucleotide sequence corresponding to the gene for an immunomodulatory protein.

In one mode, nucleotides corresponding to the 3' and 5' portions of the neuraminidase gene flank the gene encoding an immunomodulatory protein of SEQ ID NO: 5.

In other modalities, nucleotides corresponding to the 3' and 5' portions of the neuraminidase gene flank the gene encoding an immunomodulatory protein of SEQ ID NOs: 6 or 7.

An additional modality comprises a method for preparing a recombinant influenza virus, which in turn comprises the steps of (i) preparing the nucleic acid construct comprising the truncated neuraminidase gene and the immunomodulatory protein gene; (ii) exposing host cells concurrently to the construct of step (i) and to one more plasmids encoding the NP, PA, PB1, PB2, M1, NS and HA segments of influenza virus; (iii) recovering recombinant influenza virus from the supernatant.

In a particular form of the invention, said plasmids in step (ii) above encode cDNA encoding at least seven viral RNA segments of influenza A/PR8/34 (PR8) virus.

In another modality, the aforementioned plasmids from step (ii) above encode the minimum number of viral RNA segments required for viral particle synthesis. Where the said minimum number of viral RNA segments is at least 1, at least 2, at least 3, at least 4, at least 5, at least 6, or at least 7.

Also, in another modality, the method of preparing recombinant influenza virus comprises the additional steps of infecting a substrate with the recombinant influenza virus recovered in step (iii) above and purifying the virus from the substrate.

Another modality of the invention relates to an immunogenic composition comprising recombinant influenza virus and a pharmaceutically acceptable vehicle.

The said immunogenic composition is, in an alternative modality, capable of inducing long-lasting heterosubtypic immune response. Also, such a composition is preferably administered intranasally.

Finally, the invention also includes the use of influenza virus to prepare an immunogenic composition to prevent or treat infections caused by influenza viruses, coronavirus and secondary bacterial infections, to be used as an adjuvant in vaccines, or as a tool to provide topical expression of immunomodulator polypeptides in the airways.

### Brief description of the figures

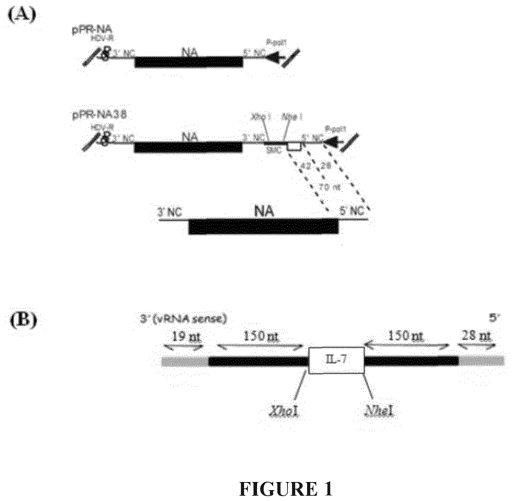

**FIGURE 1****:** Schematic representation of the construction of plasmid pPRNA166x178 and pPRNA166-IL-7-178 (A) The plasmid pPRNA166x178 is derived from plasmid pPR-NA, which contains the cDNA of the complete NA segment in negative orientation under the control of the truncated human polymerase I promoter and the hepatitis delta virus ribozyme. To obtain pPRNA166x178, first it was introduced an additional 3' promoter and an XhoI/NheI cloning site into this plasmid. The last 42 nucleotides of the NA ORF (white square) were then duplicated, to preserve the integrity of the 5' end of the neuraminidase along its last 70 nucleotides, resulting in the plasmid pPR-NA38. The NA truncation was accomplished by replacing the ORF present in the plasmid with the first 169 nucleotides and the last 178 nucleotides of the NA segment, flanking the multiple cloning site and removing the 170-1232 nucleotides. The first 166 nucleotides of the NA were then replaced with a sequence containing all the ATG truncations (nucleotides 20-22; 62-64; 115-117; 163-165) mutated to the CTA truncation. (B) pPRNA166-IL-7-178 was obtained from cloning the heterologous sequence of murine interleukin 7 (IL-7), constructed as a synthetic gene using the corresponding cDNA sequence, into the cloning site recognized by *Xho*I/*Nhe*I restriction enzymes.

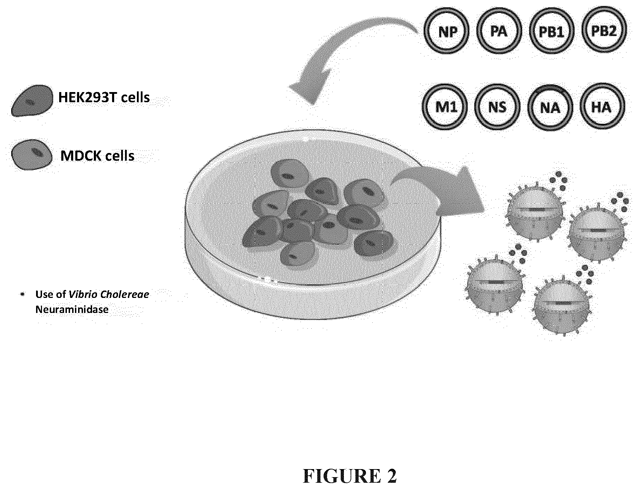

**FIGURE 2****:** Reverse genetics of influenza virus. Recombinant viruses were obtained by reverse genetics following the methodology described by Kawaoka and co-workers (Fujii Y, Goto H, Watanabe T, Yoshida T, Kawaoka Y. Proc Natl Acad Sci USA. 2003 Feb 18;100(4):2002-7), with modifications. Thus, co-cultures of HEK 293T and MDCK cells (grown in DMEM culture medium with antibiotics and supplemented with 10% SFB) were co-transfected with the plasmid encoding a segment of recombinant NA containing or not containing IL-7 (pPRNA166-IL-7-178 and pPRNA166x178, respectively) and seven other plasmids encoding the other segments of influenza virus PR8 (NP, PA, PB1, PB2, M1, NS and HA,). We used 250 ng of each plasmid in 12 µl of Fugene HD (Promega), allowing the reconstitution of 8 functional ribonucleoprotein complexes *in vivo* and thus the transcription and replication of the viral segments. Twenty-four hours after transition, the culture medium was replaced with DMEM culture medium with antibiotics and supplemented with TPCK trypsin (SIGMA) and *Vibrio cholerae* neuraminidase (SIGMA). Supernatants were collected 72 hours after transfection. After an amplification step in MDCK cells, the transfectant viruses were cloned and subsequently amplified in MDCK cells. Recombinant influenza viruses carrying the NA166x178 segment will hereafter be referred to as Flu-CT (control virus), while viruses carrying the NA166-IL7-178 segment will hereafter be referred to as Flu-IL7.

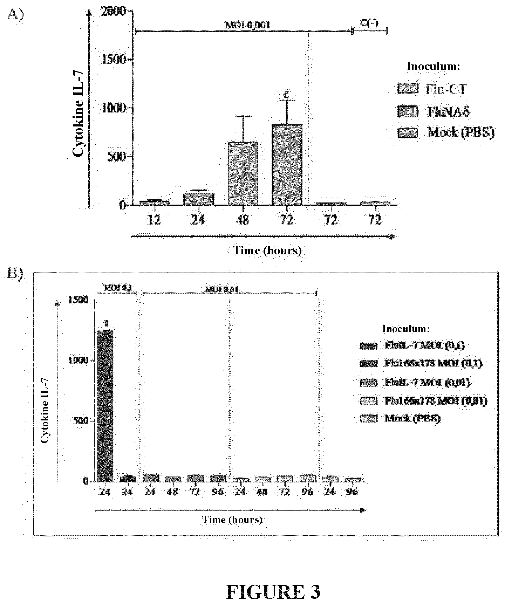

**FIGURE 3****:** Characterization of recombinant influenza viruses carrying the interleukin 7 gene in cell culture. Recombinant multiply-defective influenza viruses carrying the murine IL-7 sequence (Flu-IL7), constructed by reverse genetics, were evaluated for their ability to produce IL-7 in cell culture. (A) MDCK cell monolayers were infected with Flu-IL7 or Flu-CT viruses (m.o.i = 0.001), or PBS (Mock) in the presence of TPCK trypsin and neuraminidase from *Vibrio cholerae.* The supernatant from the kinetics was collected 8h, 24for h, 48h, 72h for measurement of IL-7 by enzymatic reaction through ELISA. The results are represented in a bar graph, where the means and standard error of the mean cytokine production at each time are plotted. On the x-axis are represented the evaluated times in hours and on the y-axis is the cytokine production in pg/mL. (B). Murine macrophages derived from bone marrow monocytes were infected with PBS (Mock) or Flu-IL7 or Flu-CT virus (m.o.i = 0.1 and 0.01). At previously determined times, the supernatants of the MDCK cell cultures and the macrophages were collected, clarified by low-speed centrifugation, and frozen at -80°C. The production of IL-7 was evaluated by ELISA technique in both cases.

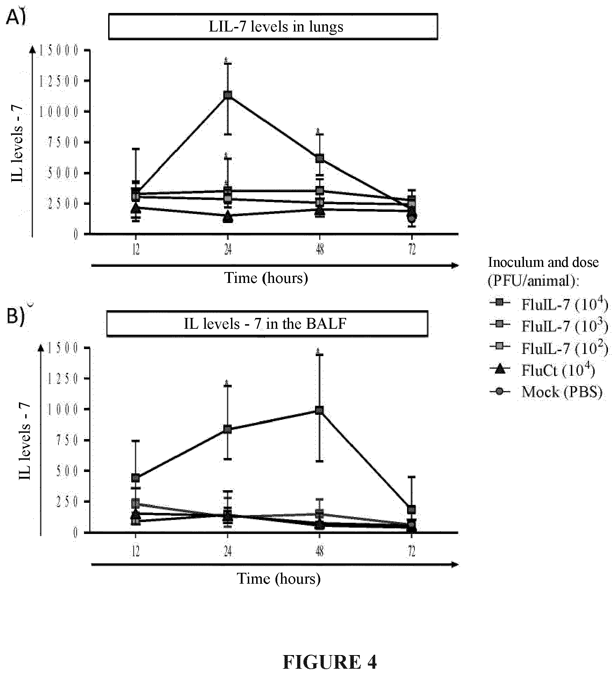

**FIGURE 4****:** Kinetics of IL-7 cytokine production in the lungs and Bronchoalveolar lavage fluid (BALF) of infected mice. The C57BL/6 mice were anesthetized and inoculated with different doses of recombinant Flu-IL7 virus (10²-10⁴ PFU/animal intranasal) or Flu-CT (10⁴ PFU/animal intranasal) in 25 µl of PBS. Animals of the mock group were inoculated only with 25 µl of PBS only. The animals were euthanized at previously determined times (12, 24, 48, and 72 hours after infection) to collect Bronchoalveolar lavage fluid and lungs and quantify the cytokine IL-7 by ELISA technique. The results of IL-7 levels (A) in the lungs and (B) in Broncho alveolar lavage fluid are shown.

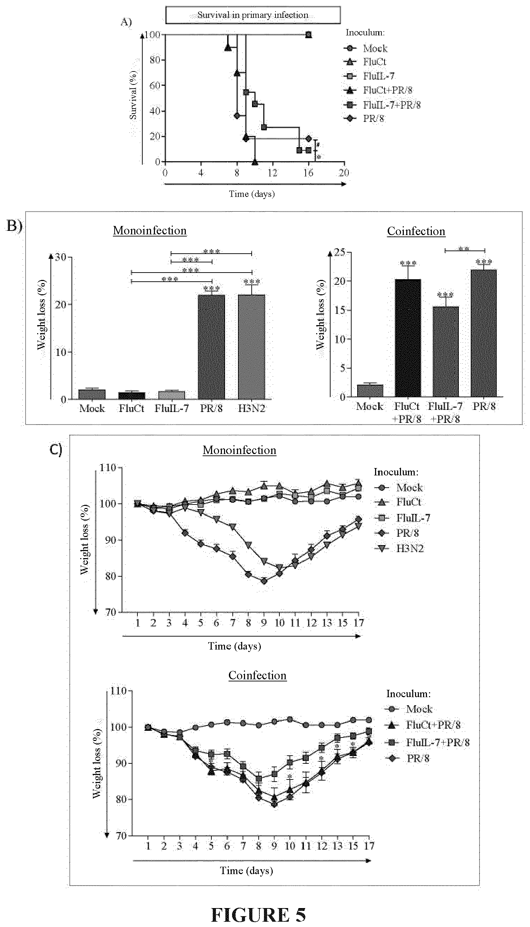

**FIGURE 5**: Recombinant influenza viruses carrying murine cytokine genes as a tool for evaluating their role in immunopathogenesis. Male C57BL/6 mice, 8-12 weeks old, were anesthetized subcutaneously with a mixture of Ketamine (100mg/kg) and Xylazine (10mg/kg) and divided into groups that were inoculated via intranasal with different viral constructs diluted in PBS in the volume of 20µL (inoculum) and followed for 17 days. (A) Mortality assessment. Intranasal inoculation was performed with PBS (Mock),¹⁰⁴ PFU (lethal dose) of PR8 or¹⁰⁴ PFU of PR8 and Flu-CT or Flu-IL7. Statistically significant differences (p <_0.05) in the *log-rank* (Mantel-Cox) and Gehan-Breslow-Wilcoxon test are represented by an asterisk (*). Differences only in the Gehan-Breslow-Wilcoxon test are represented by an octothorpe (#). (B) Total weight loss. Animals were inoculated via intranasal with PBS (Mock), 10² PFU (sub-lethal dose) of PR8 and/or 10 ⁴ PFU Flu-CT or Flu-IL7. The results are represented in *Box plot* graphs, in which the medians, 1st , and 3rd quartiles, and minimum and maximum values for each group are plotted. (C) Weight loss by time. Animals were inoculated via intranasal with PBS (Mock), 10² PFU (sub-lethal dose) of PR8, and/or 10 ⁴ PFU Flu-CT or Flu-IL7. The results are represented in symbol and line graphs, in which the means and standard error of the mean for each group/time are plotted. Statistically significant differences (p <_0.05) at each time between FluCt and FluIL-7 are represented by an asterisk (*)

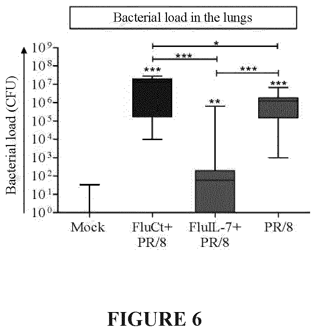

**FIGURE 6**: **Recombinant influenza virus Flu-IL7 and protection against secondary bacterial infections.** Male C57BL/6 mice, 8 to 12 weeks old, were anesthetized subcutaneously with a mixture of Ketamine (100mg/kg) and Xylazine (10mg/kg) and divided into groups (8 animals) that were immunized intranasal (40µL/animal) with 10² PFU/animal of PR8 (sub-lethal dose), PBS 1x (Mock) or co-inoculated with 10² PFU/animal of PR8 and 10 ⁴ PFU/animal of Flu-IL-7 or Flu-Ct. Ten days after infection the mice were challenged with a dose of 10² CFU/animal of (gram-positive) *Streptococcus pneumoniae* bacteria serotype ATCC6303. Forty-eight hours after the challenge, the animals were euthanized and had their lungs collected in a sterile environment for determination of the bacterial load by titration on a blood agar plate. The experiment was performed in triplicate. The data are plotted in a box diagram, in which the medians and minimum and maximum values for each group are represented on the x-axis and on the y-axis the bacterial titration values (CFU/lung). Statistically significant differences are represented by bars and asterisks, according to the degree of significance: (*) p=0.01 - 0.05, (**) p= 0.001 - 0.01 and (***) p< 0.001.

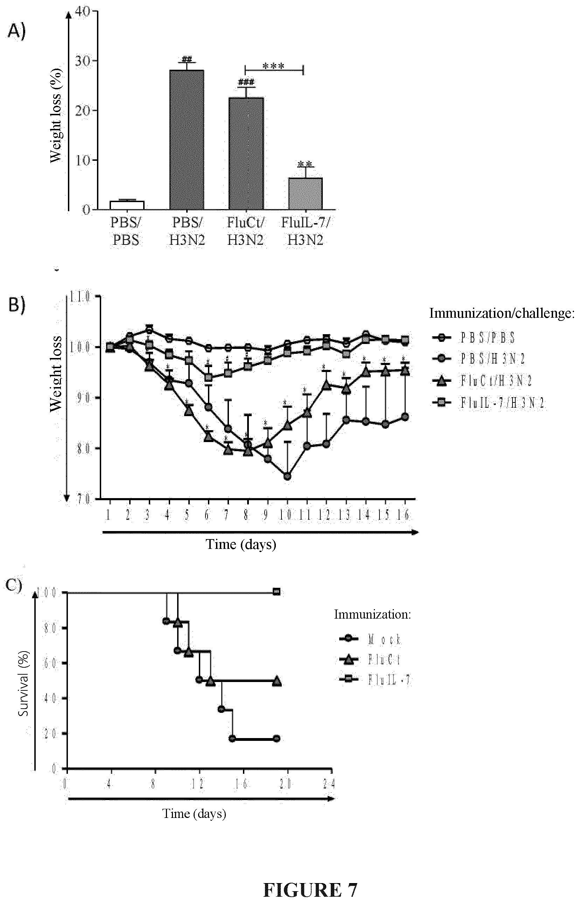

**FIGURE 7****:** Recombinant influenza virus Flu-IL7 as a tool for developing a vaccine capable of conferring heterosubtypic protection. Male C57BL/6 mice, 8 to 12 weeks old, were anesthetized subcutaneously with a mixture of Ketamine (100mg/kg) and Xylazine (10mg/kg) and divided into groups that were inoculated via intranasal injection with (i) PBS; (ii) 10⁴ PFU of Flu-CT virus; (iii) 10⁴ PFU of Flu-IL7 virus or (iv) with a sub-lethal dose (10² PFU) of an H3N2 subtype influenza virus. Two and a half months after primo-inoculation, animals were challenged with 5×10² PFU of H3N2 subtype virus or inoculated with PBS (Mock). The animals were followed for 16 days for weight evaluation. (A) Total weight loss. The results are represented in *Box plot* graphs, in which the medians, 1st and 3rd quartiles, and minimum and maximum values for each group are plotted. (B) Weight loss by time. The results are represented in symbol and line graphs, in which the means and standard error of the mean for each group/time are plotted. Statistically significant differences (p <_0.05) between FluCt immunized group and FluIL-7 immunized group is represented by astern risk (*), and differences (p <_0.05) from Mock are represented by an octothorpe (#). (C) Survival curve. Alternatively, animals were inoculated as described above and challenged after one month with a lethal dose of 10³ PFU of H3N2 subtype virus or inoculated with PBS (Mock). The results are represented in *Staircase* graphs, in which survival curves are plotted for each group. On the x-axis represented the evaluated times in days and on the y axis the percentage (%) of survival. Statistically significant differences (p <_0.05) in the *log-rank* (Mantel-Cox) and Gehan-Breslow-Wilcoxon test are represented ban y asterisk (*).

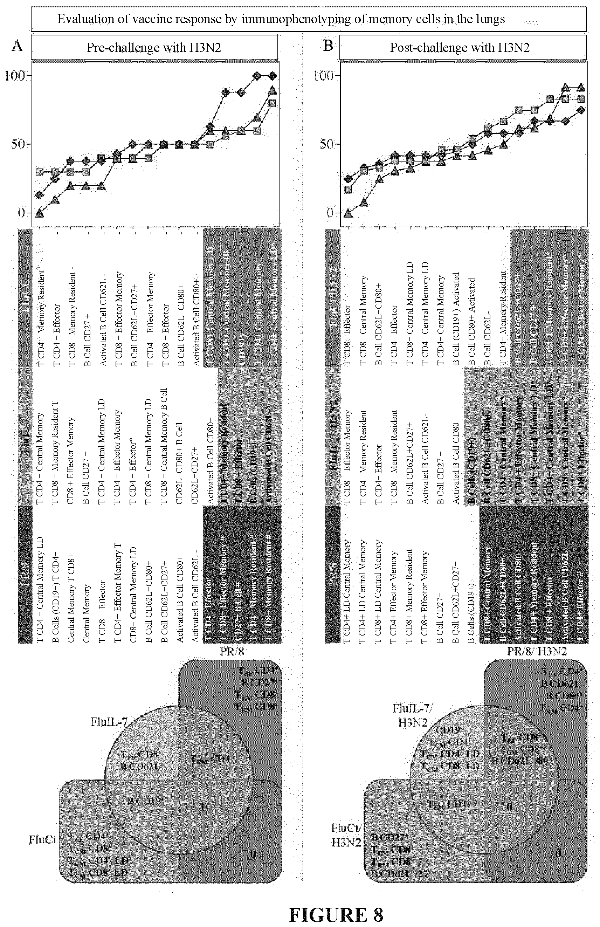

**FIGURE 8****.** Recombinant influenza virus Flu-IL7 as a tool for vaccine development capable of inducing long-lasting memory cells. Male C57BL/6 mice, 8-12 weeks old, were anesthetized subcutaneously with a mixture of Ketamine (100 mg/kg) and Xylazine (10 mg/kg) and divided into groups (6 animals) that were immunized via intranasal (40µL/animal) with¹⁰⁴ PFU of FluIL-7 or FluCt or¹⁰² PFU of PR/8 (sublethal dose) or PBS 1x (Mock). After three weeks (21 dpi) the mice were euthanized and had their lungs collected for different analyses. Other groups of animals under the same immunization scheme, were subjected after 4 weeks to a sublethal dose of 10² PFU of the H3N2 subtype virus, the mice were followed for a period of 15 days for analysis of weight loss and survival, then euthanized and had their lungs collected for different analyses. In both experiments, an *ex vivo* analysis of the phenotypic profile of effector and memory cells in the lungs was performed by flow cytometry using different biomarker panels. Different cell subpopulations and memory phenotypes were evaluated using different combinations of fluorochrome-conjugated biomarkers. : T lymphocytes (CD3+CD4+ or CD3+CD8+), effector T cells (CD44+), effector memory T cells (CD44+CD62L-CD127+) (T_{EF}), central memory T cells (CD44+CD62L+) (T_{CM})long-lived central memory T cells (CD44+CD62L+CD127+) (T_{CM}LD), resident memory T cells (CD69+CD103) (T_{RM}), B lymphocytes (CD19+), B cells memory and activation (CD62L, CD80, CD27). The "Biomarker Signatures" graphs represent the percentage of individuals with cell frequency above or below the overall median for the groups. The regions highlighted by color represent the populations that showed a frequency above the overall median of the groups in more than 50% of the evaluated individuals (ascending order). Statistically significant differences (p <_0.05) between groups are represented by an asterisk (*). The Venn diagram represents the main differences observed in each group and between groups in the "Biomarker Signatures" analysis. Statistically significant differences (p <_0.05) between the FluIL-7 and FluCt groups represented by an asterisk (*). The increase (p <_0.05) in subpopulation frequency in the PR/8 group relative to both FluIL-7 and FluCt vaccine groups represents eoctothorpehorpe (#).

### Detailed description of the invention

If not otherwise defined, all technical and scientific terms used herein have the same meaning as understood by an expert in the subject matter to which the invention belongs. Conventional molecular biology and immunology techniques are well known to a technician in the field. The narrative report also provides definitions of terms to assist in the interpretation of what is described here and the claims. Unless otherwise indicated, all figures expressing quantities, percentages and proportions, and other numerical values used in the descriptive report in claims, are to be understood as being modified in all cases by the term "about". Thus, unless otherwise stated, the numerical parameters shown in the descriptive report and the claims are approximations that may vary, depending on the properties to be obtained.

### DEFINITIONS

The terms "nucleic acid, polynucleotide, polynucleotide sequence" and "nucleic acid sequence" refer to single- or double-stranded deoxy ribonucleotide or ribonucleotide polymers or chimeras or analogues thereof. Unless otherwise indicated, a particular nucleic acid sequence of this invention comprises degenerate sequences and complementary sequences in addition to the explicitly indicated sequence.

The term "gene" is widely used to refer to any polynucleotide that encodes an instruction relating to a biological function. In general, genes comprise coding sequences and may also contain non-coding regions that regulate the expression of the coding portion. The term "gene" applies to a specific genomic sequence, a messenger RNA (mRNA) encoded by a genomic sequence, and a complementary DNA (cDNA) to an mRNA.

The non-coding regions of genes can include one or more segments that, for example, form recognition sequences for other proteins. Such non-coding segments include "promoters" and "amplifiers," for regulatory proteins such as transcription factors to bind to, resulting in the transcription of adjacent or neighboring sequences.

"Promoter" or "promoter sequence" refers to a DNA sequence whose function is to regulate the expression of a gene by initiating transcription of a nucleic acid sequence to which it is functionally linked. The regulation of gene expression can be determined in a temporal, spatial and/or physiological manner.

The term "construct" denotes a polynucleotide sequence containing at least one gene functionally linked to at least one promoter. "By functionally linked" is meant that a promoter is positioned relative to a coding sequence such that the promoter directs or regulates nucleic acid expression. Said construct can be later introduced into an expression vector. Both the construct itself and the expression vector can subsequently be introduced into a host cell to promote the expression of one or more genes.

An "expression vector" is a vector, such as a plasmid, which is capable of promoting expression, e.g. transcription, of a coding polynucleotide sequence incorporated therein. Expression vectors can be self-replicating or not self-replicating. Typically, the polynucleotide sequence to be expressed is "functionally linked" to a promoter and/or enhancer that regulates its transcription.

The term "introduced", when referring to a heterologous gene or isolated polynucleotide, refers to the incorporation of a polynucleotide into a eukaryotic or prokaryotic cell where the nucleic acid can be incorporated into the genome of the cell (e.g., chromosomal, plasmid, plastid or mitochondrial), converted to an autonomous or transiently expressed replicon (e.g., transfected mRNA). The term includes such methods as "infection," "transfection," "transformation," and "transduction." In the context of the invention, a variety of methods can be used to introduce polynucleotides into eukaryotic cells, including electroporation, lipid-mediated transfection (lipofection), microinjection, and virus-mediated transfer, etc.

The term "encode," as used herein, refers to the property of nucleic acid, for example, deoxyribonucleic acid (DNA), to transcribe a complementary nucleic acid, including a nucleic acid that can be translated into a polypeptide. For example, DNA may encode a ribonucleic acid (RNA) that is transcribed from that DNA. Similarly, DNA can encode a polypeptide translated from RNA transcribed from DNA.

The term "truncated", as used throughout this report, denotes the interruption of a polynucleotide or polypeptide chain, preventing the expected physiological functioning of said truncated sequence. For example, a truncated neuraminidase gene, as described herein, corresponds to the polynucleotide sequence that encodes neuraminidase that has been interrupted by the removal of a medial fragment of the native sequence.

The term "host cell" means a cell into which a nucleic acid has been introduced, such as a vector, and also includes progeny of that cell that also has this nucleic acid. The host cells can be prokaryotic cells, such as bacterial cells, or eukaryotic cells, such as, for example, yeast, insect, amphibian, avian, or mammalian cells.

The term "recombinant" indicates that the material (e.g., a nucleic acid or protein) has been artificially or synthetically (not naturally) modified by human intervention. Specifically, when referring to a virus, for example, an influenza virus, the virus is recombinant when it is produced by the expression of a recombinant nucleic acid.

The term "immunogenic composition" denotes a preparation that contains a substance capable of inducing a memory immune, humoral, and/or cellular response against one or more diseases. In general, the response-inducing substance, commonly called an "antigen", is usually part or all of the causative agent of the disease from which it is desired to be protected.

The ability of an antigen to induce an immune response is called "immunogenicity". For example, immunogenic composition against infections caused by different types of influenza viruses can be produced from structural proteins of the viral subtype(s) against which protection is desired. Alternatively, said vaccines can be produced with intact viruses, however, inactivated or with attenuated infectious capacity.

The immune response generated by an antigen may be specific for a disease-causing agent or may be capable of conferring protection against more than one agent. Particularly in the case of immunogenic compositions against the influenza virus, each antigen can generate a response against a single viral strain. When a single antigen is capable of generating an immune response against several strains of other influenza virus subtypes, the immune response is then termed "heterosubtypic".

A long-lasting heterosubtypic response is defined as a response that activates immunological memory, protecting against antigenically distinct influenza viruses 2 months or more after treatment.

The immune response to an antigen can be made more effective when the immunogenic composition contains one or more adjuvants. In the present context, adjuvants are substances of synthetic, organic or inorganic, or biological origin that increase the effectiveness of an immune response when administered together with an antigen.

Furthermore, immunomodulatory proteins can be part of an immunogenic composition capable of inducing a heterosubtypic immune response of the present invention. In this context, the term "immunomodulatory protein" denotes a polypeptide with regulatory biological activity on the functioning of immune cells.

Therefore, an immunomodulatory protein can be employed to restore the natural balance or to enhance the activity of a patient's immune system. As desired in the present invention, the immunomodulatory protein enhances the activity of the immune system, contributing to the response generated against the antigen. Broadly speaking, immunomodulatory proteins include chemokines and cytokines such as, for example, MIP-1 (*Macrophage Inflammatory Protein*)*,* MCP-1 (*Monocyte Chemoattractant Protein*)*,* G-CSF (*Granulocyte-colony stimulating factor*)*,* GM-CSF (*Granulocyte-Macrophage Colony-Stimulating Factor*)*,* TNF-α (*Tumor Necrosis Factor*), interferons and interleukins.

Immunogenic compositions can be formulated for any manner of administration including, for example, oral, intranasal or parenteral. The term parenteral, as used herein, includes subcutaneous, intradermal, intravascular (e.g., intravenous), intramuscular, spinal, intracranial, intrathecal, and intraperitoneal injection, as well as any similar injection or infusion technique. In certain embodiments, intranasal administration may be preferred.

### MODALITIES

The present invention represents an advance over the state of the art in providing a recombinant, multiplication-defective influenza virus capable of generating a long-lasting heterosubtypic immune response.

Without any intention to limit the inferential range of the phenomena represented here experimentally, the inventors have demonstrated that the expression of IL-7 concomitantly with exposure to the influenza virus *in vivo* in an individual confers long-lasting protection against other subtypes of said virus and bacterial infections, and can also be used in the treatment of infections caused by coronaviruses, such as, for example, coronaviruses associated with severe acute respiratory syndromes (SARS-CoV and SARS-CoV-2).

Concomitant expression of IL-7 was achieved by introducing a gene encoding such a cytokine between segments encoding the 3' and 5' portions of the viral neuraminidase. Due to the truncation of the neuraminidase gene by the removal of its medial portion, the recombinant influenza virus became multiplication defective and, therefore, unable to multiply *in vivo.*

Other strategies can be explored for the development of multiplication-defective recombinant influenza viruses. This includes the mutation of NS sequences responsible for processing messenger RNA in NS1 and NS2 (Wolschek, M. et al. Establishment of a chimeric, replication-deficient influenza A virus vector by modulation of splicing efficiency. J Virol 85, 2469-2473, 2011), deletion of the hemagglutinin (HA) protein coding sequence (Watanabe, T., Watanabe, S., Noda, T., Fujii, Y. & Kawaoka, Y. Exploitation of nucleic acid packaging signals to generate a novel influenza virus-based vector stably expressing two foreign genes. J Virol77, 10575-10583, 2003) or the PB2 protein (Uraki, R. et al. A Bivalent Vaccine Based on a PB2-Knockout Influenza Virus Protects Mice From Secondary Pneumococcal Pneumonia. J Infect Dis 212, 1939-1948, 2015). In these cases, it is necessary to use cell lines, constitutively transfected to produce viral HA or PB2 or cells that do not produce interferon (in the case of influenza virus with modified NS segment).

One skilled in the art will understand that the experiments described herein are representative of a concept that encompasses (i) a nucleic acid construct comprising a truncated neuraminidase gene into which the sequence encoding an immunomodulatory protein has been inserted; (ii) a recombinant influenza virus comprising the nucleic acid construct (i); a method for making recombinant influenza virus; (iii) an immunogenic composition; and (iv) the use of the recombinant influenza virus, as an immunogen or adjuvant, to prepare a vaccine to prevent infections caused by influenza viruses and secondary bacterial infections or as a tool to promote the topical expression of immunomodulatory polypeptides in the airways. Additionally, the invention also encompasses the use of the recombinant influenza virus in the preparation of a therapeutic vaccine in infections caused by coronaviruses.

In certain embodiments, the virus is an influenza virus of type A or B, having viral RNA (vRNA) segments derived from one or more than one precursor virus. In certain embodiments, the recombinant influenza virus is a multiplication defective virus.

In certain embodiments, the recombinant influenza virus is multiplication defective in that it has truncated neuraminidase, which is non-functional. Furthermore, the recombinant influenza virus neuraminidase is not functional due to the removal of its medial portion (nucleotides 170-1232).

In some embodiments, the heterologous protein produced by the virus is an immunomodulatory protein. In these embodiments, said immunomodulatory protein may be a chemokine. In alternative forms, the immunomodulatory protein may be a cytokine. Preferably, the immunomodulatory protein is an interleukin (IL) such as, for example, IL-15, IL-17, IL-4, IFN-G, and particularly IL-7.

For the purposes of this invention, the nucleotide sequence encoding IL-7 preferably originates an IL-7 of the same biological origin as the individual who is to be protected or treated from an influenza virus or coronavirus infection. For example, to protect a human subject, the nucleotide sequence encoding IL-7 used in the construction of the invention encodes a human IL-7. Likewise, porcine IL-7 is used to protect pigs. Illustratively, the application of the described construction is exemplified below using the murine homolog of IL-7, since, in an experimental phase of technology development, its effectiveness was evaluated in a murine model of infection by the influenza virus. In this regard, it will be apparent to the person skilled in the art to use the nucleotide sequence encoding IL-7 suitable for the desired construction.

Nucleotide sequences encoding human IL-7 or homologs, for example from mouse (murine), swine, equine, rat, or *rhesus* monkey are known to the person skilled in the art. In some species*(e.g*., humans and mice), the IL-7 gene transcript is reported to be subject to alternative post-transcriptional processing (*splicing*), resulting in more than one variant. Despite variations, the corresponding sequences can be found in public repositories, for example in GenBank, under accession numbers NM_000880.4 (*Homo sapiens;* transcriptional variant 1; SEQ ID NO: 2), NM 214135.2 (*Sus scrofa domesticus;* SEQ ID NO: 3), NM_008371.5 (*Mus musculus;* transcriptional variant 1; SEQ ID NO: 4), NM_013110 (*Ratus novergicus*)*,* NM_001032846 (*Macaca mulatta*) and XM_519820 (*Pan troglodytes;* transcriptional variant 1).

Polypeptides derived from nucleotide sequences encoding IL-7 are also available in public databases and are known to the person skilled in the art. For example, transcriptional variant 1 of the human IL-7 gene, when translated, produces a protein of 177 amino acids, with amino acids 1-25 of the N-terminal portion making up a signal peptide that is cleaved during post-processing protein translation. Thus, the mature IL-7 polypeptide corresponds to a sequence of 152 amino acids (SEQ ID NO: 5).

One skilled in the art will understand that the component of the technical effect of the present invention attributed to IL-7 is centered on the ability of IL-7 to bind to the IL-7 receptor (IL-7R) and transduce a signal through the said receptor. Therefore, it will be evident to the person skilled in the art that the nucleotide sequence that encodes an immunomodulatory protein, particularly an IL-7, can be a sequence that encodes any polypeptide capable of, in this case, interacting with and activating the IL-7R.

The present invention encompasses nucleic acid molecules containing portions encoding an IL-7, wherein said portions are nucleic acid sequences of at least 40%, 45%, 50%, 55%, 60%, 65%, 70% , 75%, 80%, 85%, 90%, 95%, 96%, 97%, 98%, 99% or 100% identical to the nucleic acid sequences described by SEQ ID NO: 2, 3 or 4. Nucleic acid molecules that are at least 95% identical to that represented by SEQ ID NO: 2 are particularly preferred.

In another embodiment, the invention makes use of nucleic acid sequences encoding an IL-7 that are at least 40%, 45%, 50%, 55%, 60%, 65%, 70%, 75%, 80 %, 85%, 90%, 95%, 96%, 97%, 98%, 99% or 100% identical to the amino acid sequences of SEQ ID NOs: 5, 6 or 7.

In a certain embodiment of the invention, the nucleotide sequence encoding an immunomodulatory protein, preferably an IL-7, is introduced into an expression vector comprising the sequence corresponding to the truncated viral neuraminidase gene. Generally, the sequence encoding the immunomodulatory protein, preferably an IL-7, is introduced between nucleotide sequences corresponding to the 3' and 5' regions of the neuraminidase gene. In particular, the sequence encoding the immunomodulatory protein, preferably an IL-7, is introduced immediately after the 166th nucleotide, 3'-5' sense, of the sequence encoding the neuraminidase.

Particularly, the vector encoding the truncated neuraminidase is pPRNA166x178 (SEQ ID NO: 1). The vector contains the ORF of said neuraminidase in negative orientation. The expression of the neuraminidase is impeded due to the removal of its medial portion (nucleotides 170-1232) and replacement of all ATG triplets from the first 166 nucleotides to the CTA triplet (nucleotides 20-22; 62-64; 115-117; 163- 165). One skilled in the art will recognize that the nucleotide sequence encoding an immunomodulatory protein, preferably an IL-7, can be introduced into plasmid pPRNA166x178 through recombinant DNA techniques, supported by *Xho*I/*Nhe*I restriction enzymes.

Thus, in a particular embodiment of the invention, the pPRNA166x178 vector, in which a nucleotide sequence encoding an IL-7 was introduced immediately after the 166th nucleotide, 3'-5' sense, of the sequence encoding the WSN virus neuraminidase, called pPRNA166-IL-7-178, corresponds to SEQ ID NO: 9.

In certain embodiments, the methods of the invention comprise introducing a plurality of vectors, each of which incorporates a portion of an influenza virus into a population of host cells capable of supporting viral replication. Host cells can be cultured under conditions permissible for viral growth, and the influenza virus can be recovered.

In certain embodiments, a method of producing recombinant viruses comprising a segmented RNA genome is provided, which method comprises the steps of a) introducing into one or more expression vectors containing the viral cDNA corresponding to each gene in the viral genome; b) introducing said expression vectors into a host cell or a population of host cells; c) incubating said host cells, and d) isolating a population of recombinant influenza viruses.

The introduction of the nucleic acid into a host cell or cell population results in the transcription of influenza virus genomic RNA, and, in the presence of suitable influenza proteins, the RNA transcript can be packaged into a multiplication-defective recombinant influenza virus.

In some embodiments, the host cell is selected from the group consisting of Vero cells, Per.C6 cells, BHK cells, PCK cells, MDCK cells, MDBK cells, 293 cells (e.g., HEK 293T cells, and COS cells. In some embodiments, a population of host cells obtained by co-cultivating a mixture of at least two of these cell lines is employed, for example, a combination of HEK 293T and MDCK cells.

In certain forms of realization, expression vectors are transfected into cells by electroporation. In certain embodiments, expression vectors are introduced into cells by transfections presence of a liposomal transfection reagent or by precipitating calcium phosphate. In certain forms of realization, the expression vectors are plasmids.

In certain embodiments, the expression vectors comprise an expression vector for the expression of all genomic RNA segments necessary for the synthesis of multiplication-deficient viral particles, expression vectors that separately encode each genomic RNA segment necessary for the synthesis of multiplication-deficient viral particles, or the corresponding messenger RNAs. In certain embodiments, the expression of each segment of genomic RNA or encoded RNA is under the control of a promoter sequence derived from a human Pol I promoter.

In some embodiments of the methods described above, the recombinant influenza viruses can be recovered from the culture of host cells that incorporate the plasmids of the influenza genome. In some embodiments, the rescue of the recombinant viruses involves exposing the host cells to cell lysis conditions. In additional embodiments, the cell lysis conditions comprise the addition of digestive enzymes and/or functional exogenous neuraminidase. Furthermore, the digestive enzyme can be trypsin and the functional neuraminidase can be derived from *Vibrio cholerae.*

The methods may further comprise a step of infecting a substrate with the recovered recombinant influenza virus and purifying the virus from the substrate.

In one embodiment, one or more expression vectors encoding influenza virus viral RNA segments necessary for the synthesis of multiplication-deficient viral particles are transfected into suitable host cells concomitantly with the nucleic acid construct of the present invention.

In a particular form of the invention, said expression vectors comprise cDNA encoding the minimum number of viral RNA segments necessary for the synthesis of viral particles.

In one embodiment, expression vectors comprising cDNA encoding at least seven influenza virus viral RNA segments (i.e., NP, PA, PB1, PB2, M1, NS, and HA) are transfected into suitable host cells concomitantly with nucleic acid construction of the present invention.

In a particular form of the invention, said expression vectors encode cDNA encoding at least seven segments of influenza A/PR8/34 (PR8) viral RNA, as described by de Wit et al (de Wit, E. et al. Efficient generation and growth of influenza virus A/PR/8/34 from eight cDNA fragments. Virus research, v. 103, n. 1-2, p. 155-161, 2004).

The invention also provides for the use of a virus obtained by the methods of any of the modalities described above to prepare an immunogenic composition for preventing infections caused by influenza viruses and secondary bacterial infections or treating infections caused by coronaviruses, either as an immunogen or as an adjuvant in combination with another immunogen or drug with antiviral activity. In addition to said recombinant influenza virus, the immunogenic compositions required herein may also include excipients and/or vehicles.

Typically, the carrier or excipient is a pharmaceutically acceptable one, which, by way of example, but not limited to, can be: sterile water, aqueous saline, buffered aqueous saline, aqueous dextrose, aqueous glycerol, ethanol, fluid allantoic from eggs from uninfected hens (i.e. normal allantoic fluid "NAF") or combinations thereof. The preparation of such solutions that guarantee sterility, pH, isotonicity and stability is carried out according to protocols established in the art.

Optionally, the formulation for the administration of influenza virus or subunits thereof also contains one or more adjuvants to enhance the immune response to influenza antigens. The arsenal of adjuvants known to the person skilled in the art includes saponin, mineral gels such as aluminum hydroxide, cell surface active substances such as lysolecithin, pluronic polyols, and polyanions, peptides, oil or hydrocarbon emulsions, bacillus Calmette-Guerin (BCG) and *Corynebacterium parvum.*

In certain embodiments, the immunogenic compositions will generally be in aqueous form. However, some compositions may be in dry form and the form of injectable solids or dry preparations or polymerized in an adhesive. Immunogenic compositions may include preservatives such as thimerosal or 2-phenoxyethanol.

In certain embodiments, the immunogenic compositions may include one or more buffers. Typical buffers include phosphate buffer, Tris, borate buffer, succinate buffer, histidine buffer (particularly with an aluminum hydroxide adjuvant), or citrate buffer. The pH of an immunogenic composition will generally be between 5.0 and 8.1, and more typically between 6.0 and 8.0 e.g. 6.5 and 7.5, or between 7.0 and 7.8. A method of the invention may therefore include a step of adjusting the pH of the bulk immunogenic composition before packaging.

The immunogenic composition may be a therapeutic composition or a prophylactic vaccine systemically administered or used as an immunotherapeutic, for example, by subcutaneous or intramuscular injection using a needle and syringe or a needleless injection device. Alternatively, the vaccine formulation is administered intranasal, by droplet, large particle aerosol (greater than about 10 microns) or spray, into the upper respiratory tract. Although any of the above routes of delivery result in a systemic immune response, intranasal administration confers the added benefit of evoking mucosal immunity at the site of influenza virus entry.

In general, the recombinant influenza viruses of the invention are administered in an amount sufficient to stimulate an immune response specific to one or more influenza virus subtypes. Preferably, administers the ration of the influenza virus evokes a protective heterosubtypic immune response.

Furthermore, the recombinant influenza viruses of the invention can be administered in an amount sufficient to stimulate a therapeutic immune response against Coronavirus infection.

Dosages and methods for evoking a protective immune response against one or more strains of influenza virus are known to those of skill in the art.

The present invention is described by the non-limiting examples below, which are merely illustrative. Various modifications and variations of embodiments are apparent to the skilled artisan, without departing from the spirit and scope of the invention.

### EXAMPLES

### EXAMPLE 1: CONSTRUCTION AND CHARACTERIZATION OF RECOMBINANT INFLUENZA VIRUSES

Plasmid pPRNA166x178 encodes a truncated neuraminidase segment in which all ATG triplets of the first 166 nucleotides of the 3' region (nucleotides 20-22; 62-64; 115-117; 163-165) have been mutated to the CTA triplet. This segment is followed by a multiple cloning site and the last 178 nucleotides of the 5' region of the neuraminidase segment. The coding sequence of the murine cytokine IL-7 was constructed as a synthetic gene by the company GENSCRIPT (Hong Kong) based on the cDNA sequence, having been optimized for expression in murine cells (SEQ ID NO: 8), without alteration. on the amino acids encoded by the sequence (SEQ ID NO: 6). The optimized sequence was cloned into plasmid pPR166x178 at the multiple cloning site, giving rise to vector pPRNA166-IL-7-178 (SEQ ID NO: 9). This type of construction allows the production of the cytokine IL-7 in its native form within the infected cell, given that the mutations in the neuraminidase segment described above prevent the production of any peptide from this protein.

Plasmid pPRNA166x178 was constructed from plasmid pPR-NA. pPR-NA is a transfer plasmid containing the cDNA encoding the wild type WSN virus neuraminidase segment. Its construction was previously described (Machado, A., N. Naffakh, S. van der Werf, and N. Escriou. 2003. Virology 313:235-249). This plasmid contains the neuraminidase segment cloned in negative orientation into transfer plasmid pPR7 between the truncated human polymerase I (pol I) promoter sequence and the hepatitis delta virus ribozyme sequence. This type of construction allows the synthesis of a synthetic viral RNA molecule in the transfected cells.

pPR-NA was successively modified until it resulted in the plasmid pPRNA166x178: (i) First, the neuraminidase 3' promoter region was amplified and inserted after the neuraminidase ORF present in the plasmid, together with a multiple *Xho*I*lNhe*I cloning site; (ii) Next, to preserve the integrity of the 5' end of the neuraminidase throughout its last 70 nucleotides, the last 42 nucleotides of the neuraminidase ORF were amplified and inserted into the 5' end of the neuraminidase already cloned into the plasmid, resulting in the plasmid pPR-NA38 (Vieira Machado A, et al. Recombinant influenza A viruses harboring optimized dicistronic NA segment with an extended native 5' terminal sequence: induction of heterospecific B and T cell responses in mice. Virology. 2006 Feb 5;345(1):73-87) (iii) The neuraminidase from plasmid pPR-NA38 was then replaced by a trunced neuraminidase (due to the removal of nucleotides 170-1232). For that, the first 169 nucleotides of the 3' region and the last 178 nucleotides of the 5' region of the neuraminidase segment were amplified from the neuraminidase sequence present in the pPR-NA plasmid. These two amplification products were successively cloned, replacing the previously cloned neuraminidase ORF and flanking the previously introduced multiple cloning site; (iv) to prevent the expression of peptides encoded by the 169 nucleotides of the neuraminidase 3' region, a synthetic gene with all the ATG triplets of the first 166 nucleotides of the neuraminidase 3' region mutated (nucleotides 20-22; 62-64; 115 -117; 163-165), constructed by the company GENSCRIPT (Hong Kong), it was cloned into the multiple cloning site of the plasmid described above, replacing the 3' region of the neuraminidase previously cloned in the plasmid, finally resulting in plasmid pPRNA166x178.

Plasmids encoding the remaining segments of the A/PR8/34 (PR8) virus were kindly provided by Dr. Ron Fouchier of the Erasmus Institute Rotterdam (Netherlands) under a material transfer agreement.

Recombinant influenza viruses were obtained by reverse genetics according to the technique shown in Figure 2 and previously described by Kawaoka et al (Fujii Y et al, Selective incorporation of influenza virus RNA segments into virus. Proc Natl Acad Sci USA. 2003 Feb 18;100(4):2002-7). Briefly, co-cultures of sub confluent monolayers of HEK 293T cells and MDCK cells grown in modified Dulbecco's medium (DMEM) in the presence of 10% fetal bovine serum and antibiotics were co-transfected with: 1) the plasmid encoding a recombinant segment of the neuraminidase, with or without IL-7 (pPRNA166-IL-7-178 and pPRNA166x178, respectively) and 2) the plasmids encoding the other (seven) segments of the influenza virus, PR8. In this way, eight ribonucleoprotein complexes were reconstituted *in vitro,* allowing the transcription and replication of all viral segments and the synthesis of new viral particles. Twenty hours after transfection, the culture medium was replaced by DMEM supplemented with 1µg/ml of trypsin TPCK (SIGMA) and 300 µU/ml of *Vibrio cholerae* neuraminidase (which makes up for the lack of viral neuraminidase, allowing the release of the neoformed viruses). After 72 hours of incubation at 37°C, supernatants from the transfected cells were collected. The recombinant viruses obtained carrying the IL-7 gene (Flu-IL7) and the respective control virus, without IL-7 (Flu-CT) were amplified and purified twice by limiting dilution (higher dilution at which we observed a cytopathic effect) in MDCK cells, before being submitted to a final amplification in this same cell line. All viral stocks thus obtained had their infectious titer determined by plate lysis titration under agarose on MDCK cells. The genotype of the recombinant viruses was evaluated by PCR and sequencing and did not show any deletion or mutation in the viral genome.

The production of IL-7 in cells infected by the Flu-IL7 virus was evaluated by the ELISA technique. To that end, MDCK cell monolayers were infected with Flu-IL7 or Flu-CT virus at the multiplicity of infection (M.O.I) of 1/1000. Cell culture supernatants were collected at different times after infection. As shown in Figure 3A, it was possible to detect the production of IL-7 in the infected cells from 48 hours after infection; the peak of production of this cytokine was detected 72 hours after infection, being around 800 pg/ml.

Macrophages play an important role as antigen-presenting cells in the respiratory tract, in addition to producing important inflammatory mediators in the antiviral defense. In order To the ability of the Flu-IL7 virus to infect macrophages, murine monocytes present in the bone marrow were differentiated into macrophages and infected with 0.1 or 0.01 M.O.I of recombinant virus, in the presence of neuraminidase. As shown in Figure 3B, IL-7 production in macrophages was detected 24 hours after infection with the Flu-IL7 virus using M.O.I 0.1. These results evidence the ability of the Flu-IL7 virus to infect macrophages, in which they are able to exprescancytokine.

### EXAMPLE 2: EVALUATION OF IL-7 PRODUCTION IN MICE INFECTED WITH THE FLU-IL7 VIRUS

Male C57BL/6/C mice between 8 and 10 weeks of age were anesthetized and inoculated intranasal with 10², 10³ or 10⁴ PFU of Flu-IL7 virus or 10⁴ PFU Flu-CT diluted in 25 µl of PBS. The Mock group was inoculated with PBS only. At 12, 24, 48, or 72 hours after inoculation, the animals were euthanized for collection of lungs and Broncho alveolar lavage (BALF). The production of IL-7 was evaluated by the ELISA technique. As shown in Figure 4A, it was possible to detect significantly increased levels of IL-7 in the lungs of animals inoculated with Flu-IL7 at a dose of 10⁴ PFU, at 24 and 48 hours after infectionhile in BALF, the peak of expression of this cytokine occurred later: 48 and 72 hours after infection (Figure 4B). No significant levels of IL-7 were detected in the serum of infected animals at any time after infection (data not shown). The set of these data demonstrates the ability of the influenza virus to infect mice and express the IL-7 sequence, which is produced locally and transiently.

### EXAMPLE 3: RECOMBINANT INFLUENZA VIRUS AS TOOLS FOR THE STUDY OF THE IMMUNOPATOGENESIS OF INFLUENZIMMUNOPATHOGENESIS

The various studies that evaluate the immunopathogenesis of influenza virus infection have the disadvantage of using *knockout* mice or using systemic inoculation of cytokines, which very likely does not reflect what occurs during a transient and localized infection at the level of the respiratory tract, what is the characteristic of influenza virus infection. In this way, the present invention allows the use of an unprecedented strategy, which consists of the construction of replication-defective recombinant influenza viruses carrying murine cytokine genes, in order to assess the role of these tolatory molecules when they are transiently produced and localized during infection by the influenza virus.

To demonstrate the potential of the Flu-IL7 virus in the study of the immune pathogenesis of influenza virus infection, the following approach was used:

Male C57BL/6 mice aged 8 to 12 weeks were anesthetized subcutaneously with a mixture of Ketamine (100mg/kg) and Xylazine (10mg/kg) and divided into groups that were inoculated intranasal with different viruses. Some groups were inoculated with wild-type virus (PR8) at a dose of¹⁰² PFU (a sub-lethal dose which, nevertheless, is capable of resulting in pneumonia in inoculated animals) to assess weight loss, while other animals were infected with¹⁰⁴ PFU of PR8 to assess Mohave infected noculation with the recombinant virus was performed at a dose of¹⁰⁴ PFU of Flu-CT or Flu-IL7, diluted in PBS in the volume of 20µL (inoculum). Weight loss and mortality were followed up for three weeks. In Figure 5A, we can see that with a high dose (¹⁰⁴ PFU) of the replicative virus (PR/8) all animals become ill, with high weight loss (about 25%) and mortality in the groups inoculated with PR/8. However, the co-inoculated animals with FluIL-7 had a significantly longer survival than in the other groups inoculated with PR/8 virus. Furthermore, although high, mortality was statistically significantly lower in coinfection with FluIL-7 than with FluCt. As shown in figure 5B, the group of animals coinfected with Flu-IL7 + PR8 showed a lower total weight loss than the group that received only PR8 (Figure 5B), and a lower weight loss at specific times compared to the PR8 group and the group coinfected with Flu-CT + PR8, and recovered their initial weight earlier than the animals in the control group (Flu-CT+PR8; Figure 5C). Taken together, our results suggest a protective role for the cytokine IL-7 and demonstrate the viability of the virus we have constructed as a tool to be used in studying the immunopathogenesis of influenza virus infection.

### EXAMPLE 4: PROTECTION AGAINST SECONDARY BACTERIAL INFECTIONS AFTER IMMUNIZATION WITH FLU-IL7 VIRUS

Male C57BL/6 mice, 8 to 12 weeks old, were anesthetized subcutaneously with a mixture of Ketamine (100 mg/kg) and Xylazine (10 mg/kg) and divided into groups (8 animals) that were immunized intranasal (40µL/animal) with¹⁰² PFU/animal of PR8 (sub-lethal dose) or co-inoculated with¹⁰² PFU of PR8 and¹⁰⁴ PFU of Flu-IL7 or Flu-CT per animal or PBS 1x (mock). Ten days after infection, the mice were challenged with a^{102CFU/animal} dose of (gram-positive) *Streptococcus pneumoniae* bacteria serotype ATCC6303. Forty-eight hours after the challenge, the animals were euthanized and had their lungs collected in a sterile environment for determination of the bacterial load by titration on a blood agar plate. The experiment was performed in triplicate.

To determine the bacterial load in the lungs, they were macerated on a cell filter sieve (70 µm) with 1.5mL of saline (0.5x). The tubes were centrifuged at 2000xg for 10 min at 4 °C, the supernatant discarded, and the bacterial precipitate resuspended in 200µL of 1× PBS. The bacteria suspension was diluted (ratio 1:10) in THY medium (Todd Hewitt Broth, 5% yeast extract) and 10µL of each dilution were applied (6 replicates) to Petri plates containing blood agar (BHI-agar with 5% defibrinated sheep blood and 8 µg/mL gentamicin). The plates were kept at 37 °C under oxygen restriction for 16-18 hours. The bacterial load was determined by counting colonies with α-hemolysis (partial hemolysis) at the lowest dilution at which they could be visually separated, averaging the replicates, multiplying by the dilution correction factor, and multiplying by the conversion factor to total volume of the macerated organ (x20).

The challenge with *Streptococcus pneumoniae* was performed 10 days after infection of the animals and the lung bacterial load assessed 48h after the challenge. This assay highlighted the protective role of the cytokine IL-7 in influenza virus infection. In addition to minimizing the aggravation caused by wild-type virus, Flu-IL7 also reduced susceptibility to secondary bacterial infection by pneumococcus. Animals infected with PR8 or coinfected with Flu-CT (Flu-CT+PR8 group) showed an elevated bacterial load after challenge, with about¹⁰⁵ to¹⁰⁷ CFU/lung. On the other hand, in the animals co-inoculated with Flu-IL7 (Flu-IL7+PR8 group) the median bacterial load was¹⁰² CFU/lung (Figure 6).

### EXAMPLE 5: FLU-IL7 VIRUS AS A TOOL FOR INDUCING AN EXTENDED SPECTRUM IMMUNE RESPONSE (HETEROSUBTYPIC RESPONSE)

Male, 8- to 12-week-old C57BL/6 mice were anesthetized and immunized with¹⁰⁴ PFU of the recombinant viruses (Flu-CT or Flu-IL7) or a sub-lethal dose of¹⁰² PFU of the wild-type H3N2 subtype virus. About seventy-five days after immunization, when the memory immune response is already established, the animals were challenged with 5×10² PFU of an H3N2 subtype influenza virus.

As expected, the animals previously inoculated with H3N2 influenza virus and challenged with this same one (H3N2-H3N2 group) did not show significant weight loss, showing the presence of homo subtopic protection (against the same viral strain as the first immunization), expected after vaccination. Animals instilled with PBS and challenged with the H3N2 virus (Mock-H3N2) were those with the greatest weight loss (average 28.0% total weight loss), which is expected because they had no prior immunity. The group of animals immunized with the Flu-CT virus showed an average of 20.5% total weight loss after challenge with the H3N2 virus (Flu-CT-H3N2 group). In contrast, in the group vaccinated with Flu-IL7 virus and challenged with H3N2 virus (Flu-IL7-H3N2), the weight loss was lower (average 6.3% weight loss) (Figure 7A and 7B).

Animals immunized with Flu-CT and challenged with the H3N2 subtype influenza virus (Flu-CT-H3N2 group) showed greater weight loss, at almost all times assessed, when compared to animals immunized with Flu-IL7 and challenged with the H3N2 subtype influenza virus (Flu-IL7-H3N2 group) (Figure 7B). Therefore, immunization with a recombinant influenza virus carrying the hemagglutinin of PR8 virus (H1N1 subtype) was able to confer protection against challenge with an H3N2 subtype influenza virus, demonstrating that Flu-IL7 is able to confer heterosubtypic protection. Furthermore, the fact that this protection was detected more than 50 days after vaccination demonstrates that there is induction of a memory immune response.

In this context, once demonstrated the ability of the FluIL-7 virus to confer protection against challenge with a sub-lethal dose of the H3N2 virus, we assessed the ability of that recombinant virus to protect animals against challenge with a lethal dose. To this end, animals were immunized as described above and challenged 30 days after vaccination with the lethal dose (¹⁰³ PFU) of H3N2 virus. The group of animals inoculated with PBS alone (Mock) showed an 80% mortality after challenge, while the group immunized with FluCt virus showed a 50% mortality (Figure 7C). However, both the animals immunized with the FluIL-7 virus and the PR/8 virus survived the challenge. These results allow us to reach the following conclusions. Immunization with FluIL-7 virus confers more robust heterosubtypic protection in immunized animals, although animals in the control group (FluCt) showed milder disease compared to the Mock group. IL-7 appears to potentiate the establishment of an immunological memory, resulting in longer-lasting immunity and increased survival of challenged animals.

### EXAMPLE 6: RECOMBINANT INFLUENZA VIRUS FLU-IL7 AS A TOOL FOR THE DEVELOPMENT OF A VACCINE CAPABLE OF INDUCING LONG-LASTING MEMORY CELLS.

Male C57BL/6 mice, 8-12 weeks old, were anesthetized subcutaneously with a mixture of Ketamine (100 mg/kg) and Xylazine (10 mg/kg) and divided into groups (6 animals) that were immunized via intranasal (40µL/animal) with¹⁰⁴ PFU of FluIL-7 or FluCt or¹⁰² PFU of PR/8 (sub-lethal dose) or PBS 1x (Mock). The immunophenotypic profile of lung cells was assessed 21 days after immunization with the recombinant FluIL-7 or FluCt viruses or the wild-type PR/8 virus. In parallel, animals previously immunized with the recombinant viruses (FluCt or FluIL-7) or the wild-type PR/8 virus and challenged after 30 days with a sub-lethal dose (¹⁰² PFU) of the heterosubtypic H3N2 isolate, as mentioned above, were euthanized two weeks post-challenge and had their lungs collected for characterization of the immunophenotypic profile of leukocytes in the tissue. This assay allows the assessment of the memory response in the lungs induced by vaccination and specific against influenza, right after the second contact with the antigen (H3N2 virus). In this way, it is possible to identify the expansion of resident memory populations, as well as those that have migrated from the spleen and/or lymph nodes to the lungs in response to the challenge.

For this, the previously perfused lungs were subjected to treatment with collagenase type IV (0.5 mg/mL) (37 °C for 40 min) to allow tissue dissociation. The organs were then macerated on a cell filter sieve (70 µm) with 5mL of RPMI (10% SFB) and the suspension centrifuged at 800xg for 7 min at 8 °C. The supernatant was discarded, and RBC lysis was performed with 9mL H2O type I (20s) under vortex stirring and isotonicity corrected with 1mL of 10x PBS. To remove cell debris from the maceration, cells were subjected to a gradient of 40% isotonic Percoll^{®} (Sigma) in RPMI medium. This solution was added (10mL) to the cells (15mL tubes), the tubes were centrifuged 800xg for 20 min at 8 °C and the supernatant was aspirated and discarded. The WBCs were washed with 3mL/tube of PBS-Wash, and centrifuged at 800xg for 7 min at 8 °C and resuspended in the remaining volume after washing.

For cell viability staining, 200µL/tube of Live/Dead dye (Live/dead Fixable Aqua Dead Cell Stain exc 405 nm) diluted 1: 1,000 in 1x PBS was added, and the tubes incubated for 15 min, protected from light, at room temperature. Cells were washed (2mL/well) with PBS-Wash, centrifuged at 550xg for 7 min at 8 °C and the supernatant discarded. The total volume of cells (~2,106 cells) was dispensed (50µL/well) into a 96-well plate (bottom U) for each of the three or four panels (~5,105 cells per panel). 10µL/well of the prepared antibody mix was added at the concentration previously standardized by titration, and the cells were incubated for 30 min at room temperature and protected from light. After the incubation period, samples were subjected to fixation with 150µL/well of the commercial lysis solution (FACS^{™} Lysing Solution -BD) diluted 1:10 in type I water for 20 min at room temperature. The plates were centrifuged at 400xg for 10 min at 18 °C and the labeled cells subjected to two washes (180µL/well) with PBS-Wash. Cells were resuspended in 100µL of 1x PBS and transferred FACS tubes before reading on the LRSFortessa cytometer (100,000 events). The data obtained in the flow cytometer were analyzed in the FlowJO software (BD).

Different cell subpopulations and memory phenotypes were evaluated using different combinations of fluorochrome-conjugated biomarkers: T lymphocytes (CD3+CD4+ or CD3+CD8+), T effectors (CD44+), T effector memory (CD44+CD62L-CD127+) (TEF), T central memory (CD44+CD62L+) (TCM) and T clong lived central memory (CD44+CD62L-CD127+) (TCMLD), T memory residents (CD69+CD103) (TRM), B lymphocytes (CD19+), memory and B cell activation (CD62L, CD80, CD27).

Infection with the H3N2 subtype influenza virus resulted in different immune response profiles in the experimental groups. In animals immunized with the FluIL-7 virus, it was possible to observe a significant increase of memory CD4+ and CD8+ T lymphocytes CD4+ and CD8+ of central memory (CD44+CD62L+) and long term central memory (CD44+CD62L+CD127+) compared to animals immunized with the control FluCt virus or PR/8 virus. In addition, the percentage of effector CD8+ T cells (CD44+) was also higher in animals immunized with FluIL-7 virus compared to those immunized with FluCt virus (Figure 8). These results demonstrate that FluIL-7 vaccination induces an increase in long-lasting memory cells (CD127+), suggesting that this cytokine acts in establishing long-lasting and persistent vaccine immunity.

## Claims

1. Nucleic acid construct, **characterized by** comprising a truncated neuraminidase gene and a gene encoding an immunomodulatory protein.

2. Construction, according to claim 1, **characterized by** the fact that immunomodulatory protein is an interleukin-7.

3. Construction according to claim 1 or 2, **characterized by** the fact that it additionally comprises a promoter region and a heterologous terminator region.

4. Construction according to claim 3, **characterized by** the fact that it comprises
(i) truncated promoter of human polymerase I;
(ii) duplication of the 3' promoter of neuraminidase;
(iii) duplication of the last 42 nucleotides of the neuraminidase ORF;
in which
(a) the ATG strands of the first 166 nucleotides of the 3' region of the neuraminidase gene have been replaced with CTA; and
(b) a sequence encoding an immunomodulatory protein inserted between the first 166 and the last 178 nucleotides of the truncated neuraminidase gene.

5. Recombinant influenza virus defective for multiplication, **characterized by** expressing an immunomodulatory protein.

6. Recombinant influenza virus defective for multiplication according to claim 5, **characterized by** the fact tthe immunomodulatory protein is an interleukin-7.

7. Method for preparing defective recombinant influenza virus for multiplication as defined in claim 5, **characterized by** the fact it comprises the steps of:
(i) prepare nucleic acid construct comprising truncated neuraminidase gene and immunomodulatory protein gene;
(ii) expose host cells concurrently to the construct of step (i) and plasmids encoding the NP, PA, PB1, PB2, M1, NS, and HA segments of influenza virus;
(iii) recover recombinant influenza virus from the supernatant.

8. Method according to claim 7, **characterized by** the fact that it additionally comprises a step of infecting a substrate with the defective recombinant influenza virus for multiplication recovered in step (iii) and purifying the virus from the substrate.

9. Immunogenic composition, **characterized by** the fact that it comprises a recombinant influenza virus defected for multiplication as defined in claim 5 and a pharmaceutically acceptable vehicle.

10. Composition, according to claim 9, **characterized by** the fact that it induces long-lasting heterosubtypic immune response.

11. Composition according to claim 9 or 10, **characterized by** the fact that it is administered by the intranasal route.

12. Use of a defected recombinant influenza virus for multiplication as defined in claim 5, **characterized in that** it is for preparing an immunogenic composition to prevent or treat infections caused by influenza viruses or SARS-CoV-2, secondary bacterial infections or to be used as an adjuvant.

13. Use according to claim 12, **characterized in that** the immunogenic composition is capable of inducing a long-lasting heterosubtypic immune response and protection or treatment against influenza virus infections, SARS-CoV-2 and secondary bacterial infections.
